# EUROPEAN PATENT APPLICATION

(11) **EP 4 850 679 A1**
(43) Date of publication of application: **09.12.2026**
(21) Application number: 25217354.7
(22) Date of filing: 20.11.2025
(51) Int. Cl.: A61K 38/12, A61K 38/17, A61K 47/64, A61K 47/69, A61P 31/04

(54) **NANOPARTICLE DRUG CARRIER AND ITS PHARMACEUTICAL COMPOSITION**

(30) Priority: 03.06.2025 US 202519227426; 04.08.2025 US 202563856794 P
(71) Applicant: Fusing Therapeutics Co., Ltd, Taipei City 110502 (TW)
(72) Inventor: Liu, Yu-Han, 110502 Taipei City (TW); Hu, Che-Ming, 110502 Taipei City (TW); Chen, Te-Li, 110502 Taipei City (TW)
(74) Representative: WENDE IP GbR

(57) **Abstract**

The present invention provides a polymyxin B (PMB)-anionic molecule (AM) nanoparticle (PMB-AM NP), wherein the polycation-to-polyanion charge ratio is 2. This NP remains stable after lyophilization and reconstitution, retaining its original properties. It enhances antimicrobial efficacy, mitigates nephrotoxicity, and preserves the structural integrity of polymyxin B. In vivo studies confirm reduced renal toxicity, while antimicrobial assessments demonstrate superior bactericidal activity against multidrug-resistant pathogens. Additionally, PMB-ASO NP protects antisense oligonucleotides (ASOs) from enzymatic degradation, enabling synergistic antimicrobial effects. This invention offers a scalable and clinically translatable approach for safer and more effective polymyxin B therapy.

## Description

### BACKGROUND OF THE INVENTION

### 1. Field of the Invention

The present invention is primarily related to biomedical, but is not limited thereto. The present invention relates in particular to a nanoparticle drug carrier.

### 2. Description of the Related Art

The global health landscape is increasingly threatened by multidrug-resistant (MDR) Gram-negative bacterial infections. Recent data indicate a significant rise in MDR pathogens, particularly carbapenem-resistant *Acinetobacter baumannii, Pseudomonas aeruginosa,* and *Enterobacteriaceae,* which are associated with severe infections such as pneumonia, bacteremia, meningitis, urinary tract infections, and skin and wound infections, leading to heightened morbidity and mortality worldwide.

In light of the diminishing efficacy of carbapenems, polymyxins have resurged as critical therapeutic agents against MDR Gram-negative bacteria. Notably, polymyxin B has become the preferred clinical choice over colistin (polymyxin E) due to its pharmacokinetic advantages; polymyxin B (PMB) is administered in its active sulfate form, ensuring immediate bactericidal activity and more predictable serum concentrations. Conversely, colistin is administered as an inactive prodrug, colistimethate sodium (CMS), which requires in vivo conversion to its active form, leading to variable and often subtherapeutic levels.

### BRIEF SUMMARY OF THE INVENTION

Nanoparticle drug delivery systems have garnered significant attention in recent years due to their potential to overcome pharmacokinetic limitations of traditional antibiotics. These platforms can enhance solubility, prolong circulation time, and enable targeted delivery, thus addressing the growing challenges associated with multidrug-resistant (MDR) pathogens. Various polymeric carriers, such as poly(lactic-co-glycolic acid) (PLGA) and polyglutamic acid (PGA), have been investigated for their biocompatibility and versatility in drug encapsulation. However, issues such as insufficient stability, low drug-loading efficiency, and unpredictable in vivo behavior remain obstacles. The present invention aims to overcome these challenges by providing a coacervate-based nanocarrier with improved performance attributes.

Polymyxin B has been formulated in various ways to enhance its therapeutic efficacy or safety profile. Despite these advancements, challenges such as nephrotoxicity and neurotoxicity persist. Developing an advanced nanocarrier system is essential to enhancing antibiotic effectiveness while ensuring a safer therapeutic profile, addressing both efficacy and toxicity concerns.

The present invention provides a PMB-anionic molecule (AM) nanoparticle (PMB-AM NP) and its pharmaceutical composition. This nanoparticle aims to improve drug stability, enhance bacterial selectivity, and significantly reduce nephrotoxicity, representing a substantial advancement in the treatment of MDR Gram-negative infections.

In one of the preferred embodiments, the PMB-AM NP has a polycation-to-polyanion charge ratio ranges from 0.12 to 105.57.

In one of the preferred embodiments, wherein the polycation-to-polyanion charge ratio ranges from 0.25 to 64.

In one of the preferred embodiments, wherein the polycation-to-polyanion charge ratio ranges from 1 to 16.

In one of the preferred embodiments, wherein the polycation-to-polyanion charge ratio is 2.

In one of the preferred embodiments, wherein the ASO is 20-mer to 40-mer single-stranded DNA.

In one of the preferred embodiments, wherein the ASO is aligned to bacteria.

In one of the preferred embodiments, wherein the bacteria is Gram-negative bacteria.

In one of the preferred embodiments, wherein the Gram-negative bacteria is multidrug-resistant (MRD) Gram-negative bacteria.

In one of the preferred embodiments, wherein the Gram-negative bacteria is *Acinetobacter baumannii, Pseudomonas aeruginosa,* or *Enterobacteriaceae.*

In one of the preferred embodiments, the PMB-AM NP has a diameter of less than 30 nm.

In another aspect, the present invention provides a pharmaceutical composition, comprising a PMB-AM NP.

In one of the preferred embodiments, the composition further comprises a pharmaceutically acceptable carrier.

In one of the preferred embodiments, the composition which is for treating Gram-negative bacteria infection.

In one of the preferred embodiments, wherein the Gram-negative bacteria is multidrug-resistant Gram-negative bacteria.

In one of the preferred embodiments, wherein the Gram-negative bacteria is *Acinetobacter baumannii, Pseudomonas aeruginosa,* or *Enterobacteriaceae.*

In another aspect, the present invention provides a manufacturing method of preparing a PMB-AM NP, comprising reducing the coacervate mixture of a PMB and an AM molecule by a high-pressure homogenizer at a shear rate of 10⁶ to 10⁷ s⁻¹.

In summary, the PMB-AM NP of present invention maintains antimicrobial efficacy, significantly improves safety by mitigating nephrotoxicity, and has superior pharmacokinetic properties compared to free PMB. The controlled coacervation process facilitates the formation of stable nanoparticles and improves bioavailability.

### BRIEF DESCRIPTION OF THE DRAWINGS

In order to make the above and other objects, features, advantages and embodiments of the present invention more readily understood, the drawings are described as follows:
FIG. 1 is the characterization of PMB-PGA coacervate formation and interaction dynamics;
FIG. 2 is the maximum tolerated dose (MTD) of NP-form and non-NP-form PMB drugs in mice;
FIG. 3 is the size, morphology, and stability analysis of PMB-PGA NP at 2:1 and 1:1 charge ratios;
FIG. 4 is the stability of PMB-PGA NP before and after lyophilization, as well as the maximum tolerated dose in mice;
FIG. 5 is the nephrotoxicity evaluation of free PMB versus PMB-PGANP;
FIG. 6 is the optimal coacervation conditions for PMB-ASO NP formation.
FIG. 7 is the stability assessment of ASOs, comparing their susceptibility to enzymatic degradation in free form versus when encapsulated within PMB-ASO NP.

The various features and components in the drawings are not drawn to actual scale in accordance with the customary mode of operation, but are drawn in such a way as to best present the specific features and components associated with the present invention. In addition, similar components and parts are referred to by the same or similar reference signs in different figures.

### DETAILED DESCRIPTION OF THE INVENTION

It should be understood that the foregoing general description and the following description are exemplary and explanatory only, and are not intended to limit the scope of the claimed invention. Certain details of one or more embodiments of the present invention are set forth in the following description. Other features or advantages of the invention will be apparent from the non-limiting list of exemplary embodiments below, as well as from the appended claims.

The present invention relates to a PMB-AM NP, which is used as a component of an antimicrobial pharmaceutical composition.

As use in this specification, the terms "PMB NP" and "PMB-PGA NP" have the same meaning, unless the context clearly indicates otherwise.

As use in this specification, the term "free PMB" means PMB do not form the NP with PGA or ASO.

As use in this specification, the term "coacervate" means an aggregate of colloidal droplets held together by electrostatic attractive forces.

As use in this specification, the term "high-pressure homogenization" refers to applying high pressure to the coacervate using a high-pressure homogenizer, forcing it to pass rapidly through a narrow gap, thereby generating shear forces that reduce the coacervate into nanoparticle.

The present invention provides a PMB-AM NP, which has a polycation-to-polyanion charge ratio range from 0.12 to 105.57.

Specifically, the PMB-AM NP is the polycation engineered via electrostatic coacervation with the polyanion, wherein the polycation is PMB, and the polyanion is PGA or ASO.

Specifically, PMB contains amine-charged groups and phenylalanine aromatic groups. The aromatic group provides aromatic-aromatic interactions and contributes to the structural stability unlike conventional coacervate systems, which typically form at net charge neutrality (zero net charge).

The amine-charged groups provide the electrostatic interaction and aromatic-aromatic interactions from phenylalanine within PMB further provide structural stabilization. The presence of phenylalanine residues within PMB induces deviation from classical coacervation behavior due to cation-π interactions, which facilitate molecular stabilization and further enhance the coacervate's structural integrity.

Many existing antibiotics are mixtures, and variations in manufacturing processes or formulation polarity can lead to changes in the component ratio, affecting therapeutic efficacy and safety. Therefore, to maintain a consistent composition can ensure the uniform distribution of active pharmaceutical ingredients and prevent compositional alterations due to formulation variations.

In the biomedical field, anionic molecule (AM) refer to negatively charged compounds under physiological conditions. It is widely used in drug formulations, solubilization, and prodrug design. Common examples include carboxylates, phosphates, sulfonates, albumin, PGA or ASO. In a more preferred embodiment, the anionic molecule is PGA or ASO.

The coacervate achieves better stability at a polycation-to-polyanion charge ratio ranging from 0.12 to 105.57. In a preferred embodiment, the charge ratio ranges from 0.25 to 64. In a more preferred embodiment, the charge ratio ranges from 1 to 16, for example, 1, 2, 3, 4, 5, 6, 7, 8 ,9, 10 ,11, 12, 13, 14, 15, 16, or any positive value from 1 to 16. In a most preferred embodiment, the charge ratio is 2.

The coacervate is dispersed into a small size nanoparticle after high-pressure homogenization. Compared to traditional non-nanoparticle drugs, it is less susceptible to aggregate due to van der Waals forces, thereby achieving more uniform dispersion and stability. The nanoparticle can avoid non-uniform distribution or aggregation in non-target organs which caused toxicity or adverse drug reaction. In a preferred embodiment, the nanoparticle diameter is less than 30 nm, for example, 5 nm, 10 nm, 15 nm, 20 nm, or, any value greater than 0 nm and less than 30 nm; in a more preferred embodiment, the nanoparticle diameter is 15-20 nm.

Specifically, the present invention reduces the coacervate by high-pressure homogenization at a shear rate of 10⁶ to 10⁷ s⁻¹ (about 20k psi); in a preferred embodiment, the range of the shear rate is 10⁴ to 10⁵, 10⁵ to 10⁶, 10⁶ to 10⁷, 10⁷ to 10⁸, or a range formed by any two different values selected from 10⁴ to 10⁸.

In one of preferred embodiment, PMB-PGANP at charge ratio has similar minimum inhibitory (MIC) and minimum bactericidal concentration (MBC) with free PMB, wherein the bacteria contain carbapenem-resistant (CR) isolates, CREC2, CREC3, CRKP3, CRPA1, CRPA2, CRPA3, CRAB1, CRAB2 or CRAB3; colistin-resistant isolates, CRAS1 or CRAS3; reference strains, *P. a.* ATCC 27853, *E. coli* ATCC 25922 or *A. b.* ATCC 17978. (EC, *Escherichia coli;* KP, *Klebsiella pneumoniae;* PA, *Pseudomonas aeruginosa;* AB, *Acinetobacter baumannii;* AS, *Acinetobacter seifertii*)

In one of preferred embodiment, PMB-PGA NP has similar composition with free PMB, where in the composition contain PMB subtypes B2, B3, B1-1 or B1.

Furthermore, the optimized 2:1 charge ratio enables efficient incorporation of ASO, enhancing targeted bacterial gene silencing while protecting ASO from enzymatic degradation.

The relationship between ASO length and off-target effects exists, and extending oligonucleotides can help reduce off-target effects. ASO sequence is a single-stranded DNA which can be precisely designed to silence specific bacterial genes while minimizing off-target effects due to the inherent bacterial selectivity of PMB-ASO NP. It works by pairing with the target mRNA sequence in a complementary manner, using the antisense mechanism to inhibit gene expression.

Compared to conventional ASO fragments, which are typically around 9-12 nt in length, PMB-ASO NP enables the use of 24 nt ASO, offering greater specificity in gene silencing while expanding its potential applications.

Through electrostatic coacervation at a 2:1 charge ratio, PMB and ASO facilitates the formation of stable PMB-ASO NPs, ensuring effective ASO complexation. This system also exhibits a potential synergistic effect between PMB and ASO-mediated gene silencing, enhancing antimicrobial efficacy.

In one of preferred embodiment, the alignment of the ASO sequence (24nt) designed based on the *A. baumannii* ATCC 19606 reference template.

Specifically, the ASO is aligned to the bacteria, where in the bacteria is grams-negative bacteria; where in the grams-negative bacteria is *Acinetobacter baumannii, Pseudomonas aeruginosa,* or *Enterobacteriaceae.*

The present invention also covers a pharmaceutical composition for treating gram-negative bacteria, the method including administering to a subject (e.g., a patient) in need thereof an effective amount of the above-described therapeutic nanoparticle.

In a preferable embodiment, the pharmaceutical composition further contains an amphiphilic stabilizer. In general, the amphiphilic stabilizer is a PEG lipid. Examples of a PEG lipid include, but are not limited to, distearoyl-sn-glycero-3-phosphoethanolamine-polyethyleneglycol (DSPE-PEG), DSPE-PEG-Maleimide, DSPE-PEG-Biotin, methoxyPEG-Cholesterol (mPEG-Cholesterol), and Cholesterol-PEG-Amine.

The term "an effective amount" refers to the amount of complexes that is required to confer a therapeutic effect on the treated subject. Effective doses will vary, as recognized by those skilled in the art, depending on the types of diseases treated, route of administration, excipient usage, and the possibility of co-usage with other therapeutic treatment.

The pharmaceutical composition can be administered parenterally, orally, nasally, rectally, topically, or buccally. The term "parenteral" as used herein refers to subcutaneous, intracutaneous, intravenous, intramuscular, intraarticular, intraarterial, intrasynovial, intrasternal, intrathecal, intralesional, or intracranial injection, as well as any suitable infusion technique.

The pharmaceutical composition can be a sterile injectable composition, which can be a solution or suspension in a non-toxic parenterally acceptable diluent or solvent, such as a solution in 1,3-butanediol. Among the acceptable vehicles and solvents that can be employed are mannitol, water, Ringer's solution, and isotonic sodium chloride solution. In addition, fixed oils are conventionally employed as a solvent or suspending medium (e.g., synthetic mono- or diglycerides). Fatty acid, such as oleic acid and its glyceride derivatives are useful in the preparation of injectables, as are natural pharmaceutically acceptable oils, such as olive oil or castor oil, especially in their polyoxyethylated versions. These oil solutions or suspensions can also contain a long chain alcohol diluent or dispersant, carboxymethyl cellulose, or similar dispersing agents. Other commonly used surfactants such as Tweens or Spans or other similar emulsifying agents or bioavailability enhancers which are commonly used in the manufacture of pharmaceutically acceptable solid, liquid, or other dosage forms can also be used for the purpose of formulation.

The pharmaceutical composition can be an oral administration, which can be any orally acceptable dosage form including capsules, tablets, emulsions and aqueous suspensions, dispersions, and solutions. In the case of tablets, commonly used carriers include lactose and corn starch. Lubricating agents, such as magnesium stearate, are also typically added. For oral administration in a capsule form, useful diluents include lactose and dried corn starch. When aqueous suspensions or emulsions are administered orally, the active ingredient can be suspended or dissolved in an oily phase combined with emulsifying or suspending agents. If desired, certain sweetening, flavoring, or coloring agents can be added.

The pharmaceutical composition can be a nasal aerosol or inhalation composition, which can be prepared according to techniques well known in the art of pharmaceutical formulation. For example, such a composition can be prepared as a solution in saline, employing benzyl alcohol or other suitable preservatives, absorption promoters to enhance bioavailability, fluorocarbons, and/or other solubilizing or dispersing agents known in the art.

Above these, its reproducibility and scalability reinforce its clinical and pharmaceutical potential, positioning PMB-AM NP as a promising candidate for next-generation antimicrobial therapeutics.

In this section, the content of the invention will be described in detail by the following embodiments. These examples are for illustrative purposes only, and those skilled in the art to which this application belongs can easily identify various modifications and variations. Therefore, the following will describe in detail various embodiments of the invention, but the invention is not limited to the embodiments set forth in this specification.

### Reagents and materials

Polymyxin B sulphate was purchased from Xellia (Pharmaceuticals, Denmark). Polyglutamic acid was purchased from Peptide Institute (Osaka, Japan). DSPE-PEG was purchased from NOF Co. (Japan). Trifluoroacetic acid (TFA) was purchased from Alfa Aesar (Heysham, England). Acetonitrile was purchased from J.T. Baker (Avantor Performance Materials, Center Valley, PA). Alexa Fluor 647 dye was purchased from Thermo Fisher Scientific (Invitrogen, Carlsbad, CA).

### Coacervate complex formation by turbidity measurements

A plate reader equipped with a UV spectrophotometer (Tecan, Infinite M1000) was employed at a wavelength of 500 nm for the turbidity measurements. None of the PMB and polyanionic peptides absorbed light at this wavelength. Turbidity was measured in absorption units (a.u.).

### Thermodynamics of coacervate formation via isothermal titration calorimetry (ITC)

Calorimetric titrations of PMB into PGAs were conducted at 25°C using a MicroCal Peaq-ITC isothermal titration calorimeter (Malvern Panalytical, UK). PGA was placed in the sample cell, while PMB was loaded in the injection syringe. Titrations were performed with 2 µL injections at 150-second intervals.

### PMB-PGANP preparation

Coacervate complexation between PMB and PGA was achieved by mixing the two components at varying ratios in water followed by visual and microscopic observation of the mixtures. In a typical preparation, 9 mg of PMB and 2.25 mg of PGA were mixed in 25 mL of water containing 360 mg of DSPE-PEG. The resulting PMB-PGA coacervate was then dispersed under a shear rate of 10⁶ to 10⁷ s⁻¹ (about 20k psi) using a high-pressure homogenizer Nanolyzer-N2 (Gogene Corporation; Hsinchu County, Taiwan). The PMB-PGA coacervate be dispersed into particles of smaller diameter, namely PMB-PGA nanoparticle. DSPE-PEG remains in the final PMB-PGA nanoparticles after mixing, homogenization, and freeze-drying.

The resulting nanocoacervates were filtered to remove free PMB, PGA, or DSPE-PEG using a 30 kDa MWCO Amicon^{®} Ultra Centrifugal Filter (Merck Millipore; County Cork, Ireland) at 1100×g for 10 min. The purified PMB-PGA nanocoacervates were lyophilized and stored at -80 °C. The purified PMB-PGA nanocoacervates were buffer-exchanged to 20% sucrose, lyophilized, and stored at -80 °C. The collected AMP nano-coacervates were evaluated for size and zeta with DLS, cryoEM imaging and HPLC for physicochemical properties, morphology, particle concentration, and AMPs encapsulation efficiency, respectively. PMB-PGA NPs were then characterized for size and zeta potential.

### High-performance liquid chromatography (HPLC) analysis

The HPLC analysis was carried out in an Agilent Technologies Series 1100 apparatus (Waldbronn, Germany). The analytical column was an Ascentis Express C18 reversed phase column (Supelco) with a particle size of 5 µm (25 cm×4.6 mm). The column temperature was kept at 25°C during the quantification. For measurement of PMB, the mobile phase consisted of 30% acetonitrile in water with 37.5 mM TFA, the wavelength of detection was set at 214 nm, and the flow rate was set at 1.0 mL/min.

### Safety assessment of MTD with free PMB or PMB-PGANPs

All animal experiments were conducted following the approval of the Institutional Animal Care and Use Committee of National Defense Medical Center. Eight-week-old BALB/c mice were procured from BioLASCO Taiwan Co., Ltd (Taipei, Taiwan). To determine the maximum tolerated dose (MTD), the mice were administered PMB or PMB-PGA NPs intravenously through the tail vein at escalating doses (n=3). The MTD was defined as the highest dose at which all tested subjects survived. The MTD will be confirmed across different batches of PMB-PGA NPs.

### PMB-PGANP stability

The PMB-PGA NP exhibits a 12-fold increase in the maximum tolerated dose (MTD) relative to free PMB, underscoring its improved safety profile. Cryo-transmission electron microscopy (Cryo-TEM) and dynamic light scattering (DLS) analyses confirm the formation of monodisperse, well-defined nanoparticles with reduced aggregation, contributing to enhance formulation stability. Additionally, encapsulation efficiency assessments demonstrate significantly higher PMB loading in the 2:1 charge ratio formulation compared to alternative ratios.

### Nephrotoxicity assessment

Balb/c mice (female, 8-week-old) were subcutaneously administered at 14.4 mg/kg in, 5 doses every 2 h to reach an accumulating total dose of 72 mg/kg (n=7). At 24 h after the last dose, mice were sacrificed the left kidney from each mouse was collected and placed in were fixed by 4% paraformaldehyde (ChemCruz, US). The kidneys were subjected to histological examination at the Taiwan Mouse Clinic. Histopathological evaluation was performed by using a semi-quantitative scoring (SQS) method.

Tubular damage was classified into three levels: mild damage characterized by dilated tubules and few casts (grade 1), severe damage with cell necrosis and numerous casts (grade 2), and cortical necrosis/infarction of tubules and glomeruli, possibly with papillary necrosis (grade 3). The extent of kidney slice damage was also scored, ranging from no damage (score 0) to over 40% damage (score 6). The conversion from each grade to the score, and the overall score requires the applicant to do the calculation.

According to the three grades of renal injury, certain criteria were elaborated to manifest how to distinguish four different grades, including grade 0. The grade 0 presented no significant renal damage but was likely accompanied by occasionally spontaneous lesions, such as mildly tubular dilation and sporadic cysts. Grade 1 presented tubular dilation with several pale tubular casts in the lower power field, and mild tubular degeneration or limited necrosis.

A prominent nucleus was a sign of functionally compensated or healthy cells rather than pyknosis and karyorrhexis. Some likely control sections were graded 1 with <1% area, which can be seen as within normal limits. The grade 2 presented marked tubular cell necrosis and numerous tubular casts. The grade 3 presented acute cortical necrosis/infarction of tubules and glomeruli with or without papillary necrosis. The affected percentages of the kidney slides were straightforward. The score reflected their area through general evaluation with a microscope: <1% score = 0, 1% to <5% = score 1, 5% to <10% score = 2, 10% to <20% = score 3, 20% to <30% = score 4, 30% to <40% = score 5, and ≥40% = score 6.

In vivo nephrotoxicity studies in murine models reveal that PMB-PGA NP significantly reduces renal toxicity, as evidenced by normalized serum blood urea nitrogen (BUN) and creatinine levels. Histopathological evaluations further confirm minimal kidney damage in PMB-PGA NP-treated subjects compared to those receiving free PMB, reinforcing the formulation's improved safety. Antimicrobial efficacy assessments against carbapenem-resistant and colistin-resistant bacterial strains indicate that PMB-PGA NP retains or surpasses the bactericidal activity of free PMB.

### Antimicrobial susceptibility testing

Antimicrobial susceptibility testing was performed by the broth dilution method according to Clinical and Laboratory Standards Institute (CLSI) guidelines. MIC is defined as the lowest concentration of antibiotics required to inhibit bacterial growth. MIC values of PMB, PMB-PGA NP and PMB-ASO NP determined using the broth microdilution method. Bacterial suspensions were adjusted to a turbidity of McFarland 0.5 ± 0.05 and cultured overnight with different concentrations (2-fold dilutions) of each treatment.

The concentrations of PMB were based on its content. Following overnight incubation, the MIC endpoint was read as the lowest antibiotic concentration completely inhibiting bacterial growth upon visual inspection and absorbance measurement, with an absorbance value of 0.1 used as the cutoff for determining bacterial growth presence. MBC, defined as the lowest antibiotic concentration required to induce a 99.9% reduction from the initial bacterial inoculum according to CLSI guidelines, was evaluated by sub-culturing solutions from clear wells onto agar plates and culturing overnight.

### ASO stability against nuclease degradation

A 1 mL solution of ASO and PMB-ASO NP, prepared at an ASO concentration of 1 ng/mL, was incubated with 10 U of S1 nuclease. At predefined time points, 100 µL of the reaction mixture was collected and immediately mixed with 10 µL of 0.5 M EDTA to chelate divalent ions and inhibit nuclease activity. The samples were then heated at 70°C for 10 min to ensure complete enzyme inactivation.

Additionally, this formulation is compatible with antisense oligonucleotides (ASOs) at a 2:1 charge ratio. The coacervate PMB-ASO nanoparticle plays a crucial role in protecting ASOs from enzymatic degradation while enabling synergistic antimicrobial effects. The ASO sequence can be specifically designed to silence bacterial genes, and due to the bacterial selectivity of PMB-PGA NP, there is minimal risk of unintended gene silencing in human applications.

### Example 1

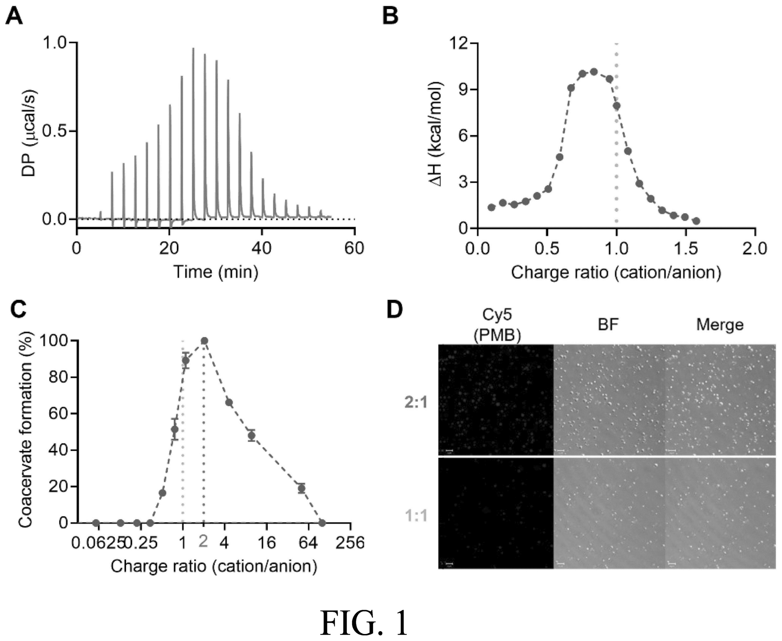

In Fig. 1A, using isothermal titration calorimetry (ITC) to analyze the thermodynamic interactions between PMB and PGA, and reveals that enthalpic contributions to coacervate formation. The result shows the indicating exothermic interactions between compound A and B, and the gradual decrease in peak height suggests saturation of binding sites over time.

The enthalpy change (ΔH) obtained by ITC reveals the thermodynamic characteristics of the interaction between A and B during the titration in the Fig. 1B. The results show that the interaction between A and B during the titration process produces a significant enthalpy change, and that this thermal change changes with the charge ratio of the cation and anion, reflecting the strength and proportionality of the bond between the two.

In Fig. 1C, the turbidity measurement result shows that coacervate has a range of formation when charge ratio (cation/anion) from 0.12 to 105.57. And it comes to the highest percentage at 2, which means 2:1 is the most preferred charge ratio for PMB-PGA NP coacervate formation.

In Fig. 1D, the fluorescence microscopy images show coacervate droplet formation at charge ratio 2:1 and 1:1, and 2:1 charge ratio appear stronger fluorescent, which means 2:1 charge ratio is more suitable for PMB-PGA NP coacervate formation than charge ratio 1:1. (Scale bar: 5 µM)

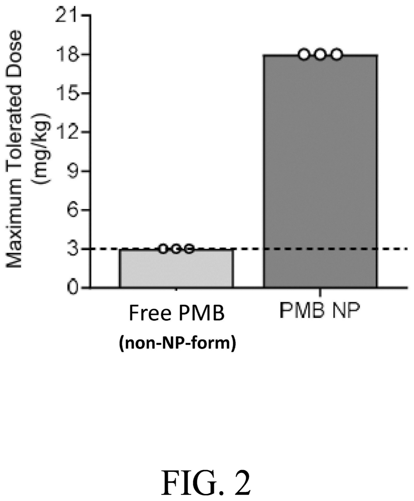

Additionally, FIG. 2 shows the MTD result that NP-form and non-NP-form PMB drugs in mice. It demonstrates that compared to non-NP-form PMB drugs, after high-pressure homogenization, the MTD of PMB NP drug is 6 times that of non-NP-form PMB drug, which means the NP-form drug is safer for organism.

### Example 2

Encapsulation efficiency (EE) is a measure of the proportion of a substance, such as drug or active pharmaceutical ingredients that has been successfully coated or encapsulated in a carrier. High EE means more effective use of drugs, and has an impact on release controlling, target delivery or stability. It is an important parameter for evaluating the quality of the manufacturing process.

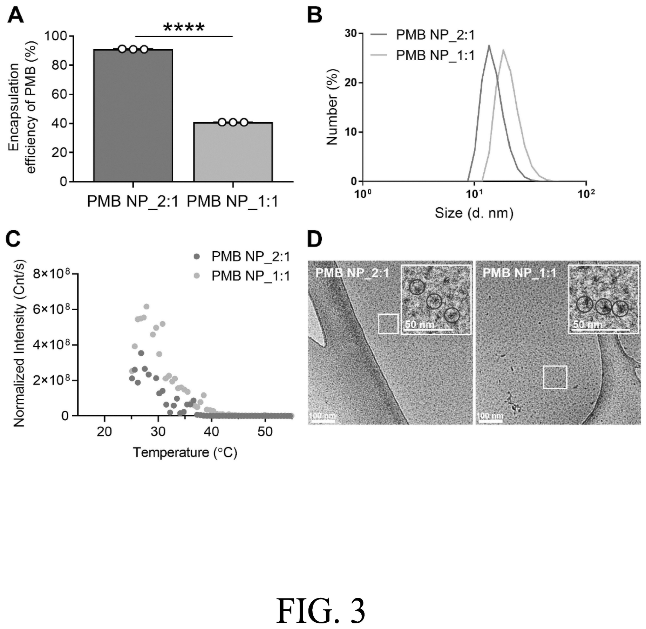

Fig. 3A and 3B show that charge ratio 2:1 has higher EE of PMB and smaller dynamic light scattering (DLS) than 1:1 in PMB-PGA NPs formulated. In the drug carrier area, that means 2:1 charge ratio has better bioavailability and stability than 1:1.

In the DLS analysis, normalized intensity (Cnt/s) is used to evaluate the light scattering change of the sample under different conditions; it indirectly reflects the stability of sample. Charge ratio 2:1 shows lower normalized intensity than 1:1 at many points under different temperatures in Fig. 3C, that means 2:1 doesn't change too drastically with temperature changes. Combines with cryo-transmission electron microscopy (Cryo-TEM) images in Fig. 3D, all of these results show that 2:1 is more stable than 1:1.

### Example 3

Table 1 presents the minimum inhibitory concentration (MIC) and minimum bactericidal concentration (MBC) values for free PMB and PMB-PGA NP formulations against various bacterial strains.

The MIC and MBC assay shows that PMB-PGA NP at charge ratio 2:1 has better antimicrobial activity than at charge ratio 1:1, that means charge ratio 2:1 can enhance the bactericidal efficacy for the nanoparticle formulations. Moreover, charge ratio 2:1 exhibits almost the same antimicrobial activity with free PMB.

**Table 1**

| **Table 1. Minimum inhibitory concentration (MIC) and minimum bactericidal concentration (MBC) of free PMB and PMB NP formulations against bacterial strains. (unit: µg/mL)** | | | | | | | |
|---|---|---|---|---|---|---|---|
| **Bacteria** | | **MIC** | | | **MBC** | | |
| | | **PMB** | **PMB NP** | | **PMB** | **PMB NP** | |
| | | | **2:1** | **1:1** | | **2:1** | **1:1** |
| **Carbapenem-resistant isolates** | **CREC 2** | **2** | **2** | **1** | **2** | **2** | **1** |
| | **CREC 3** | **2** | **2** | **2** | **2** | **2** | **2** |
| | **CRKP 3** | **2** | **2** | **4** | **8** | **2** | **>128** |
| | **CRPA 1** | **2** | **2** | **4** | **2** | **2** | **4** |
| | **CRPA 2** | **2** | **1** | **4** | **4** | **1** | **4** |
| | **CRPA 3** | **2** | **2** | **2** | **2** | **2** | **2** |
| | **CRAB 1** | **2** | **2** | **2** | **2** | **2** | **2** |
| | **CRAB 2** | **2** | **2** | **2** | **4** | **2** | **2** |
| | **CRAB 3** | **2** | **2** | **2** | **2** | **2** | **2** |
| **Colistin-resistant isolates** | **CRAS 1** | **4** | **4** | **8** | **4** | **4** | **8** |
| | **CRAS 3** | **4** | **4** | **8** | **4** | **4** | **8** |
| | **CRAN 3** | **16** | **32** | **>512** | **64** | **128** | **>512** |
| **Reference strains** | ***P.a*. ATCC 27853** | **2** | **2** | **2** | **2** | **2** | **2** |
| | ***E*. *coli ATCC* 25922** | **1** | **0.5** | **1** | **1** | **0.5** | **1** |

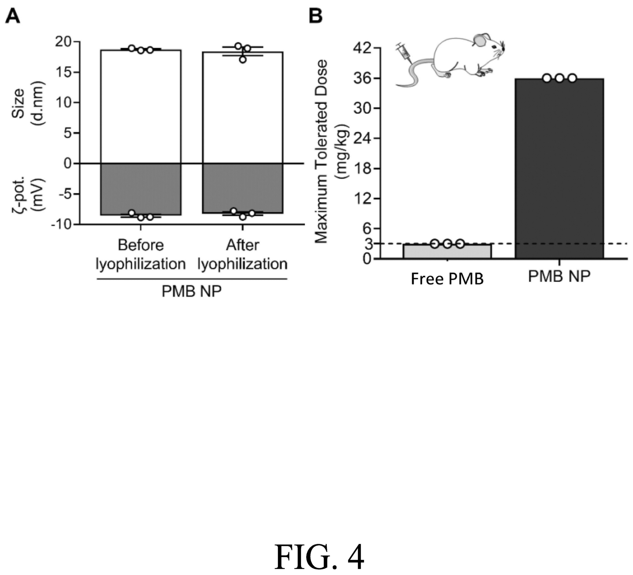

The embodiment evaluates the stability of PMB-PGA NP before and after lyophilization, as well as the MTD in mice. In Fig. 4A, stability analysis result demonstrates that PMB-PGA NP retains its physicochemical properties in size (d.nm) and zeta potential (ζ-pot., mV) following lyophilization, ensure consistent performance upon reconstitution.

In Fig. 4B, the MTD of PMB-PGA NP is 12 times that of free PMB. Combining this with the result of Table 1 above, it can be confirmed that PMB-PGA NP at charge ratio 2:1 maintains the antimicrobial activity with free PMB but the higher MTD means PMB-PGA NP significantly improved safety profile.

### Example 4

Table 2 provides a composition analysis of free PMB and PMB-PGA NP formulations, detailing the distribution of PMB subtypes (B2, B3, B1-1, B1) and impurity levels. The results confirm that PMB-PGA NP maintains the compositional integrity of free PMB, indicating that the nanoparticle formulation does not alter the fundamental structure of the antibiotic.

**Table 2**

| **Table 2. Composition analysis of free PMB and PMB NP formulations.** | | |
|---|---|---|
| **(%)** | **Free PMB** | **PMB NP** |
| **Sum (B2, B3, B1-1, B1)** | **91.5** | **91.1** |
| **B2** | **11.7** | **11.5** |
| **B3** | **3.7** | **3.6** |
| **B1-1** | **8.8** | **8.9** |
| **B1** | **67.2** | **67.0** |
| | | |
| **Sum of impurities** | **8.5** | **8.9** |

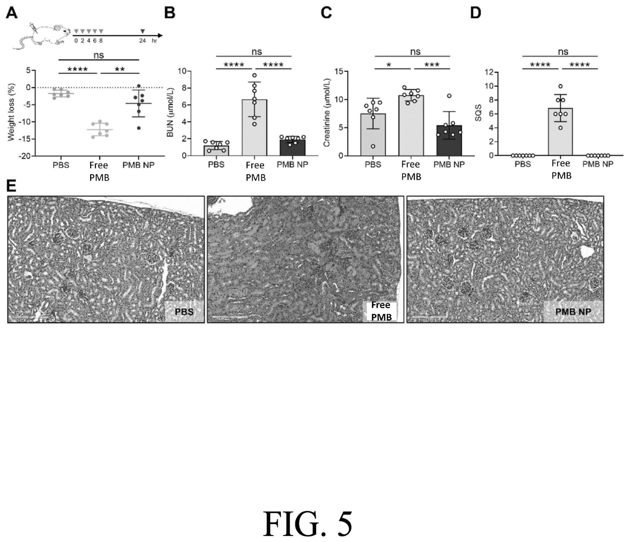

In this embodiment, the weights of mice are monitored after administrating PBS, free PMB or PMB-PGA NP. According the result of Fig. 5A, after administrating, the weight changing of PMB-PGA NP is closer to control PBS, which means PMB-PGA NP has less impact on individual.

Serum blood urea nitrogen (BUN) levels, serum creatinine and semi-quantitative scoring (SQS) as the indicator of renal dysfunction are also be monitored. In Fig. 5B to 5D, each PMB-PGA NP-treated mice show significantly lower levels than free PMB-treated mice, that demonstrates PMB-PGA NP treatment is more safer.

And the representative histological images of the kidney morphology of treated mice, proofing less renal injury in PMB-PGA NP group compared to the free PMB group (see Fig. 5E).

### Example 5

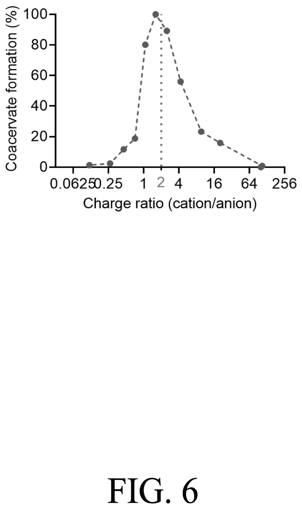

Fig. 6 shows the highest coacervate rate of PMB and ASO is the same as PMB and PGA at charge ratio 2:1, it demonstrates that PMB-AM NP charge ratio 2:1 results in efficient coacervation and stable complexation with ASO.

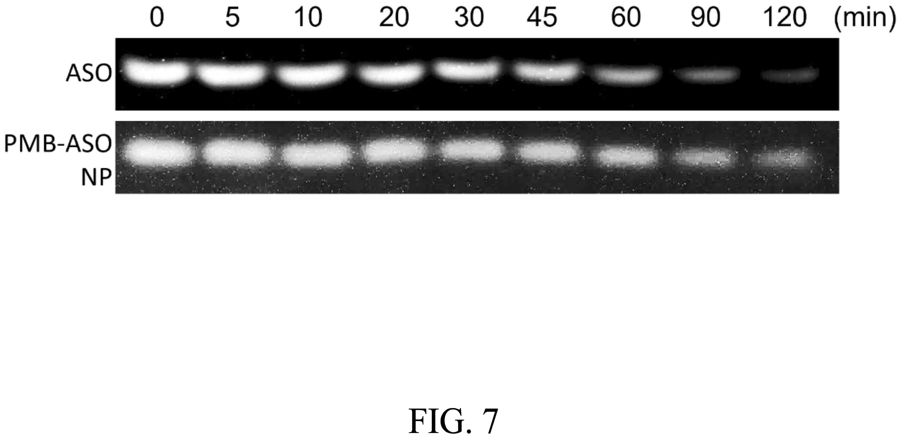

Besides, Fig. 7 presents the stability assessment of ASO, comparing their susceptibility to enzymatic degradation in free PMB versus when encapsulated within PMB-ASO NP. Upon nuclease exposure, ASOs in free form undergo degradation, whereas those incorporated within the PMB-ASO NP coacervate structure remain protected, highlighting the nanoparticle's ability to enhance ASO stability and resist enzymatic breakdown.

### Example 6

Table 3 presents the alignment of the ASO sequence (24 nt) designed based on the *A. baumannii* ATCC 19606 reference template. Sequences from different bacterial strains were analyzed for nucleotide mismatches compared to the reference.

**Table 3**

| **Table 3. Sequence alignment of ASOs in different bacterial strains and isolates.** | | | |
|---|---|---|---|
| Bacteria | Meropenem MIC (µg/mL) | Sequence (24 nt) | Mismatch (nt) |
| *A. baumannii* ATCC19606 | 0.5 | CCG**CATGGTGACTCAGCTGTTTATGAA**ACC | |
| CRAB isolate-1 | 8 | CCG**CATGGTGACTTAGCTGTTTATGA**AACC | 1 |
| CRAB isolate-2 | 128 | CCG**CATGGTGACTTAGCTGTTTATGAA**ACC | 1 |
| *E.coli* ATCC 25922 | <0.5 | CCC**CATGGTGACTCGGCGGTTTATGAC**ACG | 3 |
| *P. aeruginosa* ATCC 27853 | 0.5 | CCG**CACGGCGACACCGCGGTCTACGAC**ACC | 8 |

Table 4 details the MIC and MBC of free PMB and PMB-ASO NP formulations against bacterial strains. These sequence variations provide insight into the genetic *differences* among bacterial strains and their potential impact on antimicrobial susceptibility. The results highlight the antimicrobial activity of PMB-ASO NPs and their potential for enhanced bactericidal effects compared to free PMB.

**Table 4**

| **Table 4. Minimum inhibitory concentration (MIC) and minimum bactericidal concentration (MBC) of free PMB and PMB-ASO NP formulations against bacterial strains. (unit: µg/mL)** | | | | | | |
|---|---|---|---|---|---|---|
| Reference strains / Clinical isolates | **MIC (µg/mL)** | | | **MBC (µg/mL)** | | |
| | PMB | ASO | PMB-ASO NP | PMB | ASO | PMB-ASO NP |
| *A. baumannii* ATCC19606 | 1 | > 32 | **0.5** | 1 | > 32 | **0.5** |
| CRAB isolate-1 | 1 | > 32 | **0.5** | 1 | > 32 | **0.5** |
| CRAB isolate-2 | 1 | > 32 | **0.5** | 1 | > 32 | **0.5** |
| *E.coli* ATCC 25922 | 1 | > 32 | **0.5** | 1 | > 32 | **0.5** |
| *P. aeruginosa* ATCC 27853 | 1 | > 32 | 1 | 1 | > 32 | 1 |

The ASO sequence is aligned to grams-negative bacteria, such as *Acinetobacter baumannii, Pseudomonas aeruginosa, or Enterobacteriaceae.*

In Tables 1 and 4, compared with free PMB, the MICs of PMB-PGA NP and PMB-ASO NP remained within the range of 0.5 to 2 times, indicating that the present invention maintains similar antibacterial activity to free PMB even after forming NPs.

In summary, the embodiments of the present invention demonstrate that PMB-AM NP maintains the same excellent bactericidal activity as free PMB and exhibits lower nephrotoxicity in animal experiments, proving its potential as a drug carrier.

Although the invention has been described in the above mentioned specific embodiments, it is obvious to a person of ordinary skill that many alternatives, modifications and variations of the invention are possible. All such alternatives, modifications and variations are considered to fall within the scope of the invention.

## Claims

1. A polymyxin B (PMB)-anionic molecule (AM) nanoparticle (PMB-AM NP), which has a polycation-to-polyanion charge ratio range from 0.12 to 105.57.

2. The PMB-AM NP of claim 1, wherein the anionic molecule is poly-glutamic acid (PGA) or antisense oligonucleotide (ASO).

3. The PMB-AM NP of claim 1, wherein the polycation-to-polyanion charge ratio ranges from 0.25 to 64.

4. The PMB-AM NP of claim 3, wherein the polycation-to-polyanion charge ratio ranges from 1 to 16.

5. The PMB-AM NP of claim 4, wherein the polycation-to-polyanion charge ratio is 2.

6. The PMB-AM NP of claim 2, wherein the ASO is 20-mer to 40-mer single-stranded DNA.

7. The PMB-AM NP of claim 2, wherein the ASO is aligned to bacteria.

8. The PMB-AM NP of claim 7, wherein the bacteria is Gram-negative bacteria.

9. The PMB-AM NP of claim 8, wherein the Gram-negative bacteria is multidrug-resistant (MRD) Gram-negative bacteria.

10. The PMB-AM NP of claim 8, wherein the Gram-negative bacteria is *Acinetobacter baumannii, Pseudomonas aeruginosa,* or *Enterobacteriaceae.*

11. The PMB-AM NP of claim 1, which has a diameter of less than 30 nm.

12. A pharmaceutical composition, comprising a PMB-AM NP of claim 1.

13. The pharmaceutical composition of claim 12, further comprising a pharmaceutically acceptable carrier.

14. The pharmaceutical composition of claim 13, which is for treating Gram-negative bacteria infection.

15. The pharmaceutical composition of claim 14, wherein the Gram-negative bacteria is multidrug-resistant (MRD) Gram-negative bacteria.

16. The pharmaceutical composition of claim 14, therein the Gram-negative bacteria is *Acinetobacter baumannii, Pseudomonas aeruginosa,* or *Enterobacteriaceae.*

17. A manufacturing method of preparing a PMB-AM NP of claim 1, comprising reducing the coacervate mixture of a PMB and an AM molecule by a high-pressure homogenizer at a shear rate of 10⁶ to 10⁷ s⁻¹.
